# EUROPEAN PATENT APPLICATION

(11) **EP 1 512 664 A1**
(43) Date of publication of application: **09.03.2005**
(21) Application number: 03736169.8
(22) Date of filing: 11.06.2003
(51) Int. Cl.: C01B 13/14, C01B 33/12, A61K 7/00, A61K 7/025, A61K 7/031, A61K 7/032, A61K 7/035, A61K 7/48

(54) **POROUS METAL OXIDE MATERIAL IN FLAKE FORM, METHOD FOR PRODUCING THE SAME AND COSMETIC, COATING MATERIAL, RESIN COMPOSITION, INK COMPOSITION AND PAPER COMPRISING THE SAME**

(30) Priority: 12.06.2002 JP 2002171010
(71) Applicant: Nippon Sheet Glass Co.,Ltd., Osaka-shi, Osaka 541-8559 (JP)
(72) Inventor: YOKOI, Koji, NIPPON SHEET GLASS COMPANY, LIMITED, Osaka-shi, Osaka54 1-8559 (JP)
(74) Representative: Wilson, Peter
(86) International application number: PCT/JP2003/007466
(87) International publication number: WO 2003/106334

(57) **Abstract**

A porous metal oxide material in a flake form having a specific surface area of 110 to 3000 m²/g, an average particle diameter of 5 to 500µm, an average thickness of 0.1 to 5µm, and an average aspect ratio of 5 to 300; a method for producing the material which comprises applying a colloid solution containing colloidal particles of the metal oxide having a particle size of 5 to 500 nm on a substrate, drying to solidify the colloid solution, delaminating the resultant solid form the substrate, and subjecting the solid to a heat treatment; and a cosmetic, a coating composition, a resin composition, an ink and a paper sheet comprising the material. The porous metal oxide material in a flake formexhibits functions inherent in a porous material, is free problems such as the reduction of handlability due to coagulation, has a great mechanical strength, and exhibits great holding capability when used as a carrier.

## Description

### Technical Field

The present invention relates to a porous metal oxide material in a flake form used as a filler for a cosmetic, a coating, a resin, a film and an ink or the like, and a method for producing the same. More particularly, the present invention relates to a cosmetic, a coating composition, a resin composition, a resin molded body, an ink and paper comprising the porous metal oxide material in a flake form.

### Background Art

An inorganic material in a flake form such as mica and a glass flake is added as a filler to a coating composition, a resin composition and an ink or the like so as to improve the corrosion-resistant performance and the mechanical strength. The smoothing character or the fit feeling of the cosmetic is improved when the cosmetic comprises the inorganic material.

The porous material which has a large number of fine pores on the surface and inside can maintain various solutions or the like in the fine pores. Therefore, if the cosmetic comprises the porous material, the duration of the cosmetic is improved by adjusting oil absorption. Also, the moisture retaining property is improved by water absorption, or the durability of an odor is improved by the sustained release when an odorant is carried. The porous material functions as a carrier of a function agent such as a coloring agent as an odorant, a dye and a pigment, an antibacterial agent or a catalyst when a coating, a resin composition and an ink comprise the porous material, and various functions can be newly given thereto. When the porous material is combined with or coated on paper, the ink of a printing, a print and a writing implement or the like easily soaks into the paper, and ink dripping and ink bleeding do not occur. When the material itself has a catalyst function, the specific surface area of the material is increased by making the material into a porous body, and the catalyst function of the material is greatly improved.

Many porous materials used for the cosmetic, the coating composition, the resin composition, the ink and the paper or the like have a globular shape or irregular form (Japanese Unexamined Patent Publication No. 2001-158717, Japanese Unexamined Patent Publication No. 10-110115, Japanese Translation of International Application No. 2001-511835, and Japanese Unexamined Patent Publication No. 11-322338 or the like). Therefore, the porous materials coagulate, resulting in difficulty in handling. The porous materials have the following various problems, such as texture on skin, for instance, spreading performance is not good in the cosmetic, the coating property and moldability are worsened in the coating, the resin composition and ink, and the smoothness on the surface of the paper is deteriorated.

In an attempt to overcome these problems, a porous inorganic material in a flake form which exhibits the above various functions and does not coaguolate easily has been studied, and has already been developed. Japanese Unexamined Patent Publication No. 63-277582 discloses a method for producing the porous inorganic material in a flake form wherein a material ( for instance, a metal dissolved in an acid or an organic polymer removed by firing) capable of being removed in a solution after a flake form is formed is dispersed beforehand, after the flake form is formed, the material capable of being removed is removed from the solution.

However, a problem exists in that since the strength of the material in a flake form described in Japanese Unexamined Patent Publication No. 63-277582 is reduced, the material becomes breakable, and the material exhibits small holding capabilitywhen used as a carrier. In the method, it is necessary to disperse the material capable of being removed in the solution beforehand. Therefore, there are problems in that it takes a long process to prepare the solution, and it is difficult to select the material capable of being removed since the addition of the material capable of being removed changes the character of the solution according to the type of solution.

The present invention has been accomplished based on the above-described problems. It is an object of the present invention to provide a porous metal oxide material in a flake form which can exhibit various functions inherent in a porous material, is free from problems such as a reduction in handling performance due to coagulation, has a great mechanical strength, and exhibits great holding capability when used as a carrier, and to provide a method for producing the porous metal oxide material in a flake form at a low cost and simply. Further, it is another object of the present invention to provide a cosmetic, a coating, a resin composition, an ink and paper which effectively use various functions of the porous metal oxide material in a flake form.

### Disclosure of the Present Invention

The present inventor has conducted studies in earnest to attain the above object, and has found that a porous metal oxide material in a flake form can be easily produced by applying a colloid solution containing colloidal particles of the metal oxide on a substrate, drying to solidify the colloid solution, delaminating the resultant solid from the substrate, and heating the solid. Also, the present inventor has found that the refractive index of the porous metal oxide material in a flake form can be adjusted arbitrarily within a constant range by limiting the colloidal particles of the metal oxide to a prescribed metal oxide. Further, the present inventor has found that a powdery feeling can be effectively prevented while the availability and the moisture retaining performance or the like are improved by mixing the porous metal oxide material in a flake form with the cosmetic. When the porous metal oxide material in a flake form is mixed with the coating, the resin composition, the ink and the paper or the like, the porous metal oxide material in a flake form is free from problems such as coagulation, and is excellent in coating property, moldability, and smoothness on the surface or the like. The porous material functions as a carrier of a function agent such as a coloring agent as an odorant, a dye and a pigment, an antibacterial agent or a catalyst when the porous material is mixed with a cosmetic, coating, resin composition, ink and paper, and various functions can be newly given thereto.

### Best Mode for Carrying Out the Present Invention

Hereinafter, the embodiment of the present invention will be described in detail.

In a colloidal solution, metal oxide colloidal particles may have a nonuniform particle diameter, and preferably have an average particle diameter of 5-500 nm. When since an average particle diameter of less than 5 nm causes excessively small fine pores of the porous metal oxide material in a flake form, various functions inherent porously are not easily exhibited. On the other hand, since the average particle diameter of more than 500 nm causes excessively large fine pores, the mechanical strength of the porous metal oxide material in a flake form is reduced, and the porous metal oxide material in a flake form is not suitable for practical applications. More particularly, the average particle diameter is 10-400 nm. The gap between the colloid particles is probably fixed and formed as fine pores when the metal oxide material colloid particles are bonded to be a bulk shape.

The metal oxide colloidal particles are not limited to a particular type. However, it is preferable that the metal oxide colloidal particles primarily contain (50% by weight or more) at least one kind selected from the group consisting of silicon dioxide (SiO₂), magnesium oxide (MgO), aluminum oxide (Al₂O₃), zirconium oxide (ZrO₂), zinc oxide (ZnO), chrome oxide (Cr₂O₃), titanium dioxide (TiO₂), antimony trioxide (Sb₂O₃), and iron oxide (Fe₂O₃). Examples of components of which less than 50 % by weight may be contained in the metal oxide colloidal particles include an alkali metal oxide (Na₂O and K₂O or the like), and an alkali earth metal oxide (CaO, MgO and BaO or the like). The refractive index of SiO₂ is 1.46, the refractive index of MgO is 1.74. The refractive index of Al₂O₃ is 1.76, the refractive index of ZrO₂ is 2.20, the refractive index of Cr₂O₃ is 2.50, the refractive index of TiO₂ (anatase type) is 2.52, the refractive index of TiO₂ (rutile type) is 2.72, and the refractive index of Fe₂O₃ is 3.01. The refractive index of the porous metal oxide material in a flake form can be adjusted by appropriately selecting any one kind among the metal oxides or appropriately combining a plurality of kinds. When a plurality of kinds are combined, additivity exists. Therefore, the refractive index of the porous metal oxide material in a flake formcan be easily calculated according to the compounding ratio of the components. Commercially available fine particles having the average particle diameter and a commercially available sol solution may be used as the metal oxide colloidal particles. In addition, the metal oxide colloidal particles may be synthesized from a metal alkoxide, a metal salt, a metal organic oxide, a metal chloride or a metal nitric acid or the like.

The colloidal solution is preferably a sol constituted by dispersing the metal oxide colloidal particles with the solid content of 1-50 % by weight in the solvent. The Examples of the solvents include water, an organic solvent such as ethanol and isopropyl alcohol, and the mixture thereof. When there is a solid content of less than 1% by weight, a large amount of solvent is required and this becomes inefficient. On the other hand, a solid content of more than 50 % by weight causes excessively high viscosity of the solution.

The sol of silicon dioxide (SiO₂) can be obtained as a marketed production, for instance, Snowtex 40, Snowtex O, Snowtex C, Snowtex N, Snowtex IPA-ST, Snowtex EG-ST, Snowtex XBA-ST and Snowtex MIBK-ST (trade name, products of Nissan Chemical Industries, Ltd.), Cataloid S-30H, Cataloid SI-30, Cataloid SN, Cataloid SA, Oscal 1132, Oscal 1232 and Oscal 1332 (trade name, products of Catalysts & Chemicals Industries Co., Ltd.), Adelite AT30, Adelite AT20N, Adelite AT20A and Adelite AT20Q (trade name, products of Asahi Denka Co. , LTD.), Silicadoll 30A, Silicadoll 20A, Silicadoll 20B(trade name, products of Nippon Chemical Industries, Co., Ltd.). The sol of aluminum oxide (Al₂O₃) can be obtained as Alumina sol 100, Alumina sol 200, Alumina sol 520 (trade name, products of Nissan Chemical Industries, Ltd.), Alumina Clear Sol, Alumi sol 10, Alumi sol 20, Alumi sol SV102, Alumi sol SH5, Alumi sol CSA55, Alumi sol CSA11AD (trade name, products of Kawaken Fine Chemicals Co., Ltd.). The sol of antimony oxide (Sb₂O₃) can be obtained as A-1150,A-2550, Sun colloid ATL130, Sun colloid AMT-130 or the like (trade name, products of NissanChemical Industries, Ltd.). The sol of zirconium oxide (ZrO₂) can be obtained as NZS-30A, NZS-30B or the like (trade name, products of Nissan Chemical Industries, Ltd. ). The sol of zinc oxide (ZnO) can be obtained as Nano Tek(average particle diameter: approximately 15nm, trade name, product of C. I. Kasei Co., Ltd., content: 15% by weight, water dispersion). The sol of titanium dioxide (TiO₂) can be obtained as titania sol CS-N (average particle diameter: approximately 30 nm, anatase type, products of Ishihara Sangyo Co., Ltd.) and titanium oxide water dispersing body (average particle diameter: approximately 50 nm, rutile type, product of Ishihara Sangyo Co., Ltd.). The sol of iron oxide (Fe₂O₃) can be obtained as FRO-3W(average particle diameter: approximately 30nm, aqueous medium, Sakai Chemical Industry Co., Ltd.) and FRO-20WK(average particle diameter: approximately 200nm, aqueous medium, Sakai Chemical Industry Co., Ltd.). For instance, the sol of chrome oxide (Cr₂O₃) is obtained by pouring a chromium alum solution into a round-bottom flask, aging at 75°C and gradually generating colloidal particles in the solution.

A small amount of metal alkoxide such as tetra methoxy silicate may be added to the colloidal solution so as to help the bonding between metal oxide colloidal particles. When the metal alkoxide which is nonaqueous is added to a water medium, it is necessary to add the metal alkoxide with an organic solvent. The amount added is preferably 30 % by weight or less to the weight of the metal oxide colloidal particles. When the amount added is more than 30 % by weight, the gaps between metal oxide colloidal particles are buried by metal oxides inherent in the metal alkoxide, and the fine pores of the porous metal oxide material in a flake form are reduced. Acid or alkali as a catalyst for reactive promotion is preferably added when the metal alkoxide is added. A viscosity modifier and a surface-active agent for facilitating the application of the colloidal solution may be appropriately added to the colloidal solution.

The colloidal solution is applied on a substrate, and the coating solution is evaporated to solidify. The solidified coating film is then delaminated by a scraper or the like, and the resultant flake form is collected. A porous metal oxide material in a flake form having a large specific surface area can be obtained by heating the flake form collected at 200°C through 1200°C for 0.1-24 hours. The specific surface area can be adjusted to some degree by controlling the temperature and time of the heating. Since the synergistic effect of the temperature and time of the heat-treatment influence the shape of the porousness, that is, the optimum range of the specific surface area cannot be determined by only the temperature or only the time. The solution which remains in the fine pores is not sufficiently removed at less than 200°C, and the mechanical strength of the porous metal oxide material in a flake form obtained is reduced. On the other hand, at more than 1200°C, it is difficult to control the state of the fine pores and the specific surface area of the porous metal oxide material in a flake form obtained is less than 110m²/g since the viscous flow or the like collapses the fine pores.

Especially, the optimum heating temperature of the flake form depends on the kind of the metal oxide colloidal particle used (the melting point of the metal oxide). When silicon dioxide (SiO₂) is used, a heating temperature of 200-600°C is preferable. When titanium dioxide (TiO₂) is used, a heating temperature of 400-1000 °C is preferable. When a heat-treatment time is less than 0.1 hours, the solution which remains in the fine pores is not sufficiently removed. On the other hand, when the heat-treatment time is more than 24 hours, the manufacturing process is uneconomical. The specific surface area of the porous metal oxide material in a flake form is 110-3,000m²/g. When the specific surface area is less than 110m²/g, the porous metal oxide material in a flake form exhibits small holding capability when used as a carrier. When the specific surface area is more than 3,000m²/g, the mechanical strength of the flake form is reduced, and the flake form becomes breakable. The porous metal oxide material in a flake form has preferably a peak fine pore diameter of 2-20nm, and more preferably 3-10nm. A peak fine pore diameter of more than 20nm reduces the mechanical strength of the flake form, and the flake form becomes breakable.

After the heat-treatment, the porous metal oxide material in a flake form is pulverized and is classified and prepared to a prescribed particle diameter if necessary. The porous metal oxide material in a flake form is not limited to a particular size and shape, however, it is preferable that the porous metal oxide material in a flake form has an average particle diameter of 5 to 500µm, more particularly 8 to 300µm, an average thickness of 0.1 to 5µm, more particularly 0.2 to 2.5µm, and an average aspect ratio of 5 to 300, more particularly 8 to 200. The average particle diameter of the porous metal oxide material in a flake form can be obtained by a laser diffraction/scattering particle size distribution measuring apparatus, for instance, Microtrack 2 (manufactured by Nikkiso Co., Ltd.). The average thickness can be obtained by the simple mean value of the measurement of 50 pieces of the porous metal oxide material in a flake form by an electron microscope. The average aspect ratio can be obtained by dividing the value of the average particle diameter by the value of the average thickness.

When the porous metal oxide material in a flake form has an average particle diameter of less than 5µm, the porous metal oxide material in a flake form agglomerates and easily becomes a mass. On the other hand, the porous metal oxide material in a flake form has an average particle diameter of more than 500µm, a nonconformity is likely to occur such that the porous metal oxide material in a flake form becomes breakable mechanically. When the porous metal oxide material in a flake form has an average thickness of less than 0.1µm, a problem is likely to occur such that it is difficult to produce the material, and the material is easily broken. On the other hand, when the porous metal oxide material in a flake form has an average thickness of more than 5µm and is mixed with the cosmetic, the availability is inferior, or when the material is mixed as a filler with a coating, an irregularity is formed on the coating film, and the appearance is deteriorated. When the porous metal oxide material in a flake form has an average aspect ratio of less than 5, a defective is likely to occur such that the porous metal oxide material in a flake form agglomerates and is likely to become a mass. When the porous metal oxide material in a flake form is mixed with the cosmetic, the availability is inferior. On the other hand, when the porous metal oxide material in a flake form has an average aspect ratio of more than 300, a nonconformity is likely to occur such that the material is easily broken mechanically.

Oil absorption can be adjusted by mixing the porous metal oxide material in a flake formwith the cosmetic, and the duration of the cosmetic is improved. Also, the moisture retaining property is improvedbyusingwater absorption, and the sustained release is given by containing an odorant in the fine pores. A function agent such as a pigment, a coloring agent and a catalyst may be mixed with the cosmetic as a carrier.

In cosmetics, the porous metal oxide material in a flake form is not particularly limited, however, the content rate is preferably 0.1-95 % by weight. When the content rate is less than 0.1 % by weight, the various effects are difficult to be exhibited. On the other hand, when the content rate is more than 95 % by weight, the characteristics as cosmetics are easily deteriorated. More preferably, the content rate is 3-70 % by weight.

The porous metal oxide material in a flake form may be appropriately subjected to hydrophobic treatment according to the form of cosmetics. Examples of methods for performing the hydrophobic treatment include a treatment by a silicon compound such as methylhydrogenpolysiloxane, high viscosity silicon oil and silicon resin, a treatment by a surface-active agent such as an anion activator and a cation activator, a treatment by polymer such as nylon, polymethyl methacrylate, polyethylene, fluoroplastic and polyamino acid, a method for performing by a perf luoro radical content compound, lecithin, collagen, metal soap, oleophic oily wax, a multiple alcohol partial ester and a complete ester or the like, and the complex process thereof. However, in general, the method for performing the hydrophobic treatment of the powder is not limited to the method described above.

Other components used for cosmetics usually can be appropriately mixed with the cosmetic of the present invention if necessary in addition to the porous metal oxide material in a flake form. Examples of other components include an inorganic powder, an organic powder, a pigment, a coloring matter, an oily component, an organic solvent, a resin and a plasticizer.

Examples of inorganic powders include talc, kaolin, sericite, muscovite, phlogopite, red mica, biotite, lepidolite, vermiculite, magnesium carbonate, calcium carbonate, kieselguhr, magnesium silicate, calcium silicate, aluminum silicate, barium silicate, barium sulphate, strontium silicate, tungsten acid metal salt, silica, hydroxy apatite, zeolite, boron nitride and ceramic powder.

Examples of organic powders include a nylon powder, a polyethylene powder, a polystyrene powder, a benzoguanamine powder, a polytetrafluoroethylene powder, di-styrene benzene polymer powder, an epoxy powder or an acrylic powder.

Examples of pigments include an inorganic white pigment such as titaniumdioxide and zinc oxide, an inorganic red pigment such as iron oxide (Bengala) and titanic acid iron, an inorganic brown pigment such as γ iron oxide, an inorganic yellow pigment such as yellow iron oxide and yellow ocher, an inorganic black pigment such as black iron oxide and carbon black, an inorganic purple pigment such as mango violet and cobalt violet, an inorganic green pigment such as chromiumoxide, hydroxide chrome and cobalt titanate, an inorganic blue pigment such as ultramarine blue and Prussian blue, a pearl pigment such as titanium dioxide coating mica, titanium dioxide coating oxy bismuth chloride, oxy bismuth chloride, titanium dioxide coating talc, fish scale foil and coloring titanium dioxide coating mica, and a metal powder pigment such as an aluminum powder and a copper powder.

Examples of coloring matters include an organic pigment such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401 and Blue No. 404, an organic pigment such as zirconium, barium or aluminium lake of Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, Blue No. 1, and a natural pigment such as chlorophyl and β-carotene.

Examples of oily components include various hydrocarbons such as squalane, liquid paraffin, vaseline, micro crystaline wax, ozokerite, ceresin, myristic acid, palmitinic acid, stearic acid, oleic acid, isostearic acid, cetyl alcohol, hexadecylic alchol, oleyl alcohol, 2-ethyl hexane acid cetyl, palmitic acid-2-ethylhexyl, myristic acid 2-octyldodecyl, di-2-ethyl hexane acid neopentyl glycol, tri-2-ethyl hexane acid glycerol, oleic acid-2-octyldodecyl, isopropylmyristate, triisostearate glycerol, tri coconut oil fatty acid glycerol, olive oil, avocado oil, beeswax, myrisryl myristate, mink oil, lanolin, esters of silicon oil, higher fatty acid and, oils and fats, higher alcohol and wax.

An organic solvent such as acetone, toluene, butyl acetate and ester acetate, a resin such as alkyd resin and urea resin, a plasticizer such as camphor and acetyl tributyl citrate, a ultraviolet absorber, an antioxidant, an antiseptic, a surface active agent, a moisturizing agent, odorant, water, alcohol or a thickener or the like may be appropriately mixed with cosmetics.

The cosmetic of the present invention is not limited to a particular form, and may be a powder form, a cake form, a pencil form, a stick form, an ointment form, a liquid form, a milky lotion form or a creamy form. Examples of the cosmetics include a facial cosmetic such as skin lotion, milky lotion and cream, and a makeup cosmetic such as foundation, lipstick, eye shadow, blusher, eyeliner, nail enamel or mascara.

The porous metal oxide material in a flake form can be used as a filler such as a coating, resin molded article (bulk and film), ink and paper. The porous metal oxide material in a flake form itself can be used as a catalyst, and as a carrier for the function agent such as an odorant, coloring agent, antibacterial agent or catalyst or the like.

### EXAMPLE

Hereinafter, the present invention will be described in detail based on the Examples, but the present invention is not limited to the following Examples.

### (Measuring method)

- Specific surface area: A specific surface area meter (NOVA1000 manufactured by Yuasa Ionics Inc.) was used. The specific surface area was measured by the BET method, and the pore distribution was measured by the BJH method. The fine pore diameter of the peak was read from the pore distribution, and was defined as the peak fine pore diameter.
- Refractive index: The refractive index was measured by an immersion method.
- Oil absorption: The oil absorption was measured according to oil absorption, pigment test method 21 of JIS K5101-1991.

### (Examples 1-4)

500g of colloidal silica having an average particle diameter of approximately 20nm (Silicadoll 30A, water dispersion, solid content rate: approximately 30% by weight manufactured by Nippon Chemical Industrial Co., Ltd.), 160g of ethanol, 30g of silicon methoxide (n-methyl silicate manufactured by Tama chemicals Co., Ltd.) and 150g of water were uniformly mixed in a stirrer. The resultant solution was cured at 50°C for 12 hours to produce a silica sol solution (colloidal solution). The silica sol solution was applied on a 10cm-dice stainless plate by a dipping method such that the average thickness after being dried was 1.0µm. After the stainless plate was placed in a drying furnace of 120°C for 5 minutes and the coating film was dried, the coating film was rubbed and delaminated by a scraper, and the flakes were collected. The flakes were placed in an alumina crucible. The flakes were sintered at a temperature and time shown in Table 1 to produce a porous metal oxide material in a flake form. The porous metal oxide material in a flake form was pulverized in a mortar, and the average particle diameter was set to approximately 10µm (average thickness of 1µm, average aspect ratio of 10).

### (Examples 5-6)

350g of colloidal silica having an average particle diameter of approximately 20nm (Silicadoll 30A, water dispersion, solid content rate: approximately 30% by weight manufactured by Nippon Chemical Industrial Co., Ltd.), 150g of titania colloid(titania sol CS-N manufactured by Ishihara Sangyo Kaisha, Ltd.), 120g of isopropanol, 30g of silicon methoxide (n-methyl silicate manufactured by Tama Chemicals Co. , Ltd.) and 150g of water were uniformly mixed in a stirrer. The compound was cured for 18 hours at 50°C to produce a silica-titania sol solution (colloidal solution). The silica-titania sol solution was applied on a 10cm-dice stainless plate by a dipping method such that the average thickness after being dried was 1.0µm. After the stainless plate was placed in a drying furnace of 120°C for 5 minutes and the coating film was dried, the coating film was rubbed and delaminated by a scraper, and the flakes were collected. The flakes were placed in an alumina crucible. The flakes were sintered at the temperature and time shown in Table 1 to produce the porous metal oxide material in a flake form. The porous metal oxide material in a flake form was pulverized in a mortar, and the average particle diameter was set to approximately 10µm (average thickness 1µm, average aspect ratio 10).

Table 1 shows the measurement results of Examples 1-6. The oil absorption of each Example shows the value of 120ml/100g or more, and it is found that the oil absorption is large. Additionally, it is found that the oil absorption tends to increase with the increase in the specific surface. Therefore, the oil absorption of the porous metal oxide material in a flake form can be arbitrarily set to some degree, and the oil absorption according to the characteristics of a cosmetic can be set by adjusting the sintering temperature and the sintering time. The porous metal oxide material in a flake form has a fine pore diameter of various sizes, and is useful as a carrier for various function materials.

**TABLE 1**

| Example | 1 | 2 | 3 | 4 | 5 | 6 |
|---|---|---|---|---|---|---|
| Sintering temperature (°C) | 400 | 400 | 600 | 600 | 400 | 800 |
| Sintering time (Hr) | 0.5 | 20 | 0.5 | 15 | 0.5 | 0.5 |
| Specific surface area (m²/g) | 220 | 210 | 200 | 180 | 240 | 130 |
| Peak fine pore diameter (nm) | 4.8 | 4.8 | 5.0 | 4.7 | 5.0 | 4.0 |
| Refractive index | 1.30 | 1.30 | 1.31 | 1.34 | 1.44 | 1.55 |
| Oil adsorption volume (ml/100g) | 200 | 195 | 190 | 180 | 140 | 120 |

### (Comparative Examples 1-2)

To 490g of silicon methoxide as a metal alkoxide (manufactured by Tama Chemicals Co., Ltd.), 500g of ethanol, 800g of water and an adequate amount of nitric acid for setting the pH of the solution to approximately 2 were added, and were uniformly mixed in a stirrer. Afterwards, the resultant solution was cured at 50°C for 8 hours to produce a coating solution, and silica flakes were produced in the same manner as the above Examples 1-6 by using the coating solution. The specific surface area, the peak fine pore diameter and the oil absorption of the silica flakes were measured by the above means. Table 2 shows the measurement results.

**TABLE 2**

| Comparative Examples | 1 | 2 |
|---|---|---|
| Sintering temperature (°C) | 400 | 800 |
| Sintering time (Hr) | 0.5 | 0.5 |
| Specific surface area (m²/g) | 20 | 5 |
| Peak fine pore diameter (nm) | 0.3 | less than 0.1 |
| Oil adsorption volume (ml/100g) | 50 | 35 |

The specific surface area of the silica flake form is 20 or less, and is remarkably smaller than those of the porous metal oxide materials in flake forms of Examples 1-6. Since the peak fine pore diameter is also 0.3 nm or less and it is difficult for a carried object to enter, the silica flake form is not useful as a carrier. The oil absorption of the silica flake form is 50 or less, and it is found that the oil absorption is also extremely small.

### (Example 7)

530g of silica colloidal having an average particle diameter of approximately 20nm (Silicadoll 30A, water dispersion, solid content rate: approximately 30% by weight manufactured by Nippon Chemical Industrial Co., Ltd.), 40g of titania particles having an average particle diameter of 250nm (CR-50 rutile type manufactured by Ishihara Sangyo Kaisha, Ltd.), 150g of ethanol and 150g of water were uniformly dispersed and mixed by using bead mills to produce a colloidal solution containing colloidal particles of silicon dioxide and colloidal particles of titanium dioxide. The colloidal solution was applied on a 10cm-dice stainless plate by a dipping method such that the average thickness after being dried was set to 2.0µm. After the stainless plate was placed in a drying furnace of 120°C for 5 minutes and the coating film was dried, the coating film was rubbed and delaminated by a scraper, and the flakes were collected. The flakes were sintered at 400°C for 1 hour to produce a porous metal oxide material in a flake form of silica-titania composite type. The porous metal oxide material in a flake form was pulverized in a mortar, so that the average particle diameter was approximately 50µm (average aspect ratio 25). The porous metal oxide material in a flake form had a specific surface area of 350 m²/g, a peak fine pore diameter of 7.2nm, a refractive index of 1.52, and an oil absorption of 270ml/100g.

### (Example 8)

470g of silica colloidal having an average particle diameter of approximately 20nm (Silicadoll 30A, water dispersion, solid content rate: approximately 30% by weight manufactured by Nippon Chemical Industrial Co., Ltd.), 60g of iron oxide particles having an average particle diameter of 60nm (FRO-6 manufactured by Sakai Chemical Industry Co., Ltd.), 150g of ethanol and 150g of water were uniformly dispersed and mixed by using bead mills to produce a colloidal solution containing colloidal particles of silicon dioxide and colloidal particles of iron oxide. The colloidal solution was applied on a 10cm-dice stainless plate by a dipping method such that the average thickness after being dried was 0.5µm. After the stainless plate was placed in a drying furnace of 120°C for 5 minutes and the coating film was being dried, the coating film was rubbed and delaminated by a scraper, and the flakes were collected. The flakes were sintered at 400°C for 1 hour to produce a porous metal oxide material in a flake form of silica- iron oxide composite type. The porous metal oxide material in a flake form was pulverized in a mortar, so that the average particle diameter was approximately 20µm (average aspect ratio 40). The porous metal oxide material in a flake form had a specific surface area of 270 m²/g, a peak fine pore diameter of 5.5nm, a refractive index of 1.71, and an oil absorption of 320ml/100g.

Next, porous metal oxide materials in flake forms produced by Examples 1-8 or silica flakes form produced by Comparative Examples 1-2 were mixed with a cosmetic, and the availability was evaluated. An evaluation of the cosmetic is performed by 10 panelists, and is a sensory evaluation of 5 stages concerning the following 5 items; spreading performance; adhesion; powdery feeling; moisture retaining feeling; duration of the cosmetic (cosmetic deterioration in four hours after the cosmetic is applied on the skin). In the evaluation results of the following Examples and Comparative Examples, the evaluation results of 10 panelists are averaged, the mean value is divided into the following five ranks, and is indicated by a sign which corresponds to each rank.
- ⓞ: 4.5 or more and 5.0 or less
- ○: 3.5 or more and less than 4.5
- ●: 2.5 or more and less than 3.5
- Δ: 1.5 or more and less than 2.5
- ×: 1.0 or more and less than 1.5

### (Example 9: Powder foundation)

A powder foundation was prepared at the component compounding ratios shown below.
(1) titanium dioxide: 7
(2) talc: 20
(3) muscovite: 3
(4) the porous metal oxide material in a flake form of Example 1: 55
(5) nylon powder: 2
(6) red iron oxide: 0.5
(7) yellow iron oxide: 1
(8) black iron oxide: 0.1
(9) silicone oil: 1
(10) palmitate-2-ethylhexyl: 9
(11) sorbitan sesquioleate: 1
(12) antiseptic: 0.3
(13) odorant: 0.1 (% by weight)

The components (1)-(8) were mixed in a henschelmixer. The components (9)-(13) which were heated, dissolved and mixed were added and mixed with the mixture. The resultant mixture was then crushed by a pulverizer. The mixture was then pressed under a pressure of 160kg/cm² in a pan having a diameter of 5.3mm to prepare a powder foundation.

### (Comparative Example 3: Powder foundation)

A powder foundation was prepared in the same way as Example 9 except that the porous metal oxide material in a flake form of the component (4) was changed into the silicon oxide in a flake form of Comparative Example 1.

### (Example 10: Solid white powder)

A white powder was prepared at the component compounding ratios shown below.
(1) talc: 10.0
(2) kaolin: 5.0
(3) titanium dioxide: 5.0
(4) zinc myristate: 5.0
(5) magnesium carbonate: 5.0
(6) sericite: 15.0
(7) the porous metal oxide material in a flake form of Example 3: 50.0
(8) coloring pigment: proper quantity
(9) squalane: 3.0
(10) tri-isooctane acid glycerin: proper quantity
(11) antiseptic, antioxidant: proper quantity
(12) odorant: proper quantity (% by weight)

The components (1) and (8) were mixed well in a blender. The components (2)-(7) were added to the mixture and mixed well. The components (9)-(11) were then added to the resultant mixture, the component (12) was then atomized to the mixture, and mixed uniformly. The mixture was then crushed with a pulverizer, and was pressed in a pan.

### (Comparative Example 4: Solid white powder)

A white powder was prepared in the same way as Example 10 except that the porous metal oxide material in a flake form of the component (7) was changed into a silicon oxide in a flake form of Comparative Example 2.

### (Example 11: Powder spray)

A powder spray was prepared at the component compounding ratios shown below.
(1) aluminium chloro hydrate: 30.0
(2) the porous metal oxide material in a flake form of Example 2: 20.0
(3) talc on which silicone treatment is applied: 15.0
(4) triclosan: 0.1
(5) isopropyl myristate: 21.9
(6) dimethylpolysiloxane: 10.0
(7) sorbitan fatty acid ester: 3.0
(8) odorant: proper quantity (% by weight)

The components (1)-(8) mixed were placed in an aerosol container. A valve was attached to the aerosol container, and a propellant was filled.

### (Comparative Example 5: Powder spray)

A powder spray was prepared in the same way as Example 11 except that the porous metal oxide material in a flake form of the component (2) was changed into silicon oxide in a flake form of Comparative Example 2.

### (Example 12: Oily stick foundation)

An oily stick foundation was prepared at the component compounding ratios shown below.
(1) the porous metal oxide material in a flake form of Example 5: 13.0
(2) titanium dioxide: 7.0
(3) kaolin: 20.0
(4) talc: 2.0
(5) mica: 3.3
(6) red iron oxide: 1.0
(7) yellow iron oxide: 3.0
(8) black iron oxide: 0.2
(9) solid paraffin: 3.0
(10) microcrystalline wax: 7.0
(11) petrolatum: 15.0
(12) dimethylpolysiloxane: 3.0
(13) squalane: 5.0
(14) isopropyl palmitate: 17.0
(15)antioxidant: proper quantity
(16) odorant: proper quantity (% by weight)

The components (9)-(15) were dissolved at 85°C. The components (1)-(8) were then added to the solution. After the solution was mixed by a dispenser, the solution was dispersed by colloid mills. The component(16) was then added to the solution. The solution was poured in a container at 70°C after deairing and cooled to prepare an oily stick foundation.

### (Comparative Example 6: Oily stick foundation)

An oily stick foundation was prepared in the same way as Example 12 except that the porous metal oxide material in a flake form of the component (1) was changed into the silicon oxide in a flake form of Comparative Example 2.

Table 3 shows the results of the sensory evaluation of Examples 9-12 and Comparative Examples 3-6.

**TABLE 3**

| | spreading performance | adhesion | powdery feeling | moisture retaining feeling | duration of the cosmetic |
|---|---|---|---|---|---|
| Example 9 | ○ | ⓞ | ⓞ | ⓞ | ⓞ |
| Example 10 | ○ | ⓞ | ⓞ | ⓞ | ⓞ |
| Example 11 | ○ | ⓞ | ⓞ | ○ | ⓞ |
| Example 12 | ○ | ⓞ | ⓞ | ○ | ⓞ |
| Comparative example 3 | ○ | ○ | Δ | Δ | ○ |
| Comparative example 4 | ○ | ○ | Δ | Δ | ○ |
| Comparative example 5 | ○ | ○ | Δ | Δ | ○ |
| Comparative example 6 | ○ | ○ | Δ | Δ | ○ |

It is found that the cosmetics of Examples 9-12 are superior in spreading performance, adhesion, powdery feeling, moisture retaining feeling, duration of the cosmetic, and the function of the porous metal oxide material in a flake form is effectively exhibited as shown in Table 3.

### (Example 13: Emulsification foundation)

An emulsification foundation was prepared at the component compounding ratios shown below.
(1) stearic acid: 0.4
(2) isostearic acid: 0.3
(3) 2-ethyl hexane acid cetyl: 4
(4) liquid paraffin: 11
(5) polyoxyethylene (10) stearylether: 2
(6) talc: 8
(7) pigment: 4
(8) cetyl alcohol: 0.3
(9) antiseptic: 0.07
(10) the porous metal oxide material in a flake form of Example 7: 10
(11) triethanolamine: 0.42
(12) propylene glycol: 5
(13) antiseptic: 0.02
(14) ion-exchange water: 54.19
(15) odorant: 0.3 (% by weight)

After the components (1)-(9) were heated, dissolved and mixed at 85°C, the component (10) was added to the mixture, and the resultant mixture was dispersed uniformly. The mixture which was heated, dissolved and mixed at 85°C was gradually added to the components (11)-(14) to emulsify the mixture. After the mixture emulsified it was stirred while the temperature at the time of emulsifying was maintained for 10 minutes, the mixture was cooled to 45°C while stirring. The component (15) was added to the mixture, and stirred and cooled up to 35°C. The product was taken out, and the product was filled in the container to prepare an emulsification foundation. Table 4 shows the evaluation results of the emulsification foundation.

**TABLE 4**

| | spreading performance | adhesion | powdery feeling | moisture retaining feeling | duration of the cosmetic |
|---|---|---|---|---|---|
| Example 13 | ○ | ⓞ | ⓞ | ⓞ | ⓞ |

### (Example 14: Blusher)

A blusher was prepared at the component compounding ratios shown below.
(1) kaolin: 19.0
(2) the porous metal oxide material in a flake form of Example 5: 5.0
(3) bengala: 0.3
(4) red No. 202: 0.5
(5) ceresin: 15.0
(6) petrolatum: 20.0
(7) liquid paraffin: 25.0
(8) isopropyl myristate ester: 15.0
(9) antioxidant: proper quantity (% by weight)

The components (1) - (4 ) were added to a part of the component (7), and the resultant mixture was processed by a roller (pigment part). On the other hand, the component (4) was dissolved in a part of component (10) (dye part). The components (5)-(9) were heated to 90°C, and dissolved. The dye part was added to the mixture, and was dispersed uniformly by a homo mixer. The mixture was filled to a prescribed container after being dispersed to prepare a blusher. Table 5 shows the evaluation results.

**TABLE 5**

| | spreading performance | adhesion | powdery feeling | moisture retaining feeling | duration of the cosmetic |
|---|---|---|---|---|---|
| Example 14 | ○ | ⓞ | ⓞ | ⓞ | ⓞ |

### (Example 15: Lipstick)

A lipstick was prepared at the component compounding ratios shown below.
(1) hydrocarbon wax: 20
(2) candelilla wax: 3
(3) glycerylisostearate: 40
(4) liquid paraffin: 26.8
(5) titanium dioxide: 4
(6) the porous metal oxide material in a flake form of Example 8: 4
(7) organic pigment: 2
(8) odorant: 0.2 (% by weight)

The components (1)-(4) were heated to 85°C and were dissolved. The components (5)-(7) were added to the mixture and were mixed while stirring. The component (8) was then mixed while stirring, and the mixture was filled in a container to prepare a lipstick. Table 6 shows the evaluation results.

**TABLE 6**

| | spreading performance | adhesion | powdery feeling | moisture retaining feeling | duration of the cosmetic |
|---|---|---|---|---|---|
| Example 15 | ○ | ⓞ | ⓞ | ⓞ | ⓞ |

### (Example 16: Eye shadow)

An eye shadow was prepared at the component compounding ratios shown below.
(1) talc: 21
(2) muscovite: 20
(3) the porous metal oxide material in a flake form of Example 6: 40
(4) pigment: 12
(5) squalane: 4
(6) cetyl-2-ethylhexaethylhexanoate: 1.9
(7) sorbitan sesquioleate: 0.8
(8) antiseptic: 0.1
(9) odorant: 0.2 (% by weight)

The components (1)-(4) were mixed in a henschelmixer. The components (5) - (9) which were heated and mixed were sprayed on the mixture. The mixture was mixed, crushed, and then pressed in a pan to prepare an eye shadow. Table 7 shows the evaluation result.

**TABLE 7**

| | spreading performance | adhesion | powdery feeling | moisture retaining feeling | duration of the cosmetic |
|---|---|---|---|---|---|
| Example 16 | ○ | ⓞ | ⓞ | ⓞ | ⓞ |

### (Example 17: Coating)

The porous material in a flake form of Example 5 was soaked to a solution of 1:1 ratio of ethanol to hinokitiol (included in hiba oil produced in Aomori and Taiwan hinoki oil, a crystalline material with bactericidal antibacterial action), and the hinokiol solution was impregnated in the porous body. The porous material in a flake form was dried at 80°C after being filtered, and the ethanol was removed. The hinokitiol was recrystallized in the fine pores. 15.6 parts by weight of the porous material in a flake form on which the hinokiol was carried, 20.6 parts by weight of alkyd resin system varnish, 10.6 parts by weight of melamine resin system varnish, and 15.6 parts by weight of swazol (trade name, aromatic system high boiling point solvent manufactured by Maruzen Petrochemical Co., Ltd. ) were dispersed for 60 minutes by using a paint shaker to produce a dispersed vehicle. In addition, 26.3 parts by weight of alkyd resin system varnish and 11.3 parts by weight of melamine resin system varnish were added and stirred to the dispersed vehicle to produce a coating composition. When the coating composition was applied on a stainless substrate, the aroma of the hinokiol (fragrance of hinoki) was fragrant. It was found that the hinokiol carried on the porous material in a flake form was reliably contained in the coating. As a result, the coating with antibacterial could be produced.

### (Example 18: Resin composition)

Rhodamine B as a basic dye was dissolved in water to produce a coloring solution of 1%. The porous material in a flake form of Example 1 was impregnated in the coloring liquid, and was dried at 80 °C after being filtered to produce a coloring material in a flake form carrying the rhodamine B. 2 % by weight of the coloring material in a flake form carrying the rhodamine B and 98% by weight of methylmethacrylate copolymerization beads were stirred and mixed in a henschelmixer to obtain a resin composition. When an acrylic resin molded article having the average thickness of 0.5mm was produced by an extrusion machine by using the resin composition, it was presented red with a beautiful bluish tone.

### (Example 19: Ink composition)

Indigocarmine (Blue No. 2) as an acid dye was dissolved in water to prepare an aqueous solution of 2%. The porous material in a flake form of Example 2 was impregnated in the solution, and was dried at 80°C after being filtered to produce a coloring material in a flake form carrying tartrazine. 19 parts by weight of ketone resin, 59 parts by weight of ethanol, and 10 parts by weight of propylene glycol monomethyl ether were mixed and stirred in 12 parts by weight of the coloring material in a flake form carrying indigocarmine to produce an ink composition. When the ink composition was used, the writing presented translucent blue.

### (Example 20: Paper)

A liquid object which consists of 75 parts of porous material in a flake form of Example 7 and 25 parts of polyvinyl alcohol was applied on the one side of a good quality paper by a bar coater such that the dry coating quantity is 20g/m², and was dried for 2 minutes at 120°C to form an ink receiving layer. A back coating layer which consists of 85 parts of light calcium carbonate (Brilliant S15 manufactured by Shiraishi Kogyo) and 15 parts by weight of styrene butadiene system latex (SN-307 manufactured by Sumika ABS Latex) was applied on the other side by a bar coater such that the dry coating quantity is 10g/m², and was dried at 120°C for 2 minutes. As a result, a record paper for an ink jet having the ink receiving layer formed on one surface and the back coating layer formed on the other surface was produced. The ink receiving layer plays a role in the rapid absorption fixation of ink, and the back coating layer plays a role in the warp prevention of paper, the strength improvement and the friction reduction in a printer or the like. The surface of the paper was flat. When the printing was performed on the surface (ink receiving layer) of the paper by using an ink jet printer, the absorption of ink was good, and the ink bleeding did not occur. In addition, the image was very sharpness.

### Industrial Applicability

The present invention exhibits the following effects by employing the compositions. The present invention provides a porous metal oxide material in a flake form which is free from problems such as a reduction in handlability due to agglomeration, has a great mechanical strength, and exhibits great holding capability when used as a carrier. Since the method of the present invention uses a colloid solution containing colloidal particles of the metal oxide having a proper average particle diameter, the porous metal oxide material in a flake form can be produced easily and simply. The state of the fine pores of the porous metal oxide material in a flake form can be appropriately adjusted by appropriately adjusting the heat-treatment condition (sintering temperature and sintering time) after the colloidal solution is molded in a flake form. The refractive index of the porous metal oxide material in a flake form can be freely designed within a constant range by appropriately selecting and combining kind of metal oxide colloidal particles. The availability of cosmetics, that is, spreading performance, moisture retaining feeling, duration of the cosmetic or the like are improved, and the oil absorption can be moderately adjusted by blending the porous metal oxide material in a flake formwith cosmetics. The optical effect such as the mat feeling and the brilliance feeling can be adjusted while the powdery feeling of the cosmetic can be prevented by controlling the refractive index of the porous metal oxide material in a flake form. The porous material functions as a carrier of a function agent such as a coloring agent such as an odorant, a dye and a pigment, an antibacterial agent and a catalyst when the porous material is blended with a coating, a resin composition and an ink, and various functions can be newly given thereto. When the porous material is blended with a paper or is coated on a paper, the ink of a printing, a print and a writing implement or the like easily soaks into the paper, and ink dripping and ink bleeding do not occur.

## Claims

1. A porous metal oxide material in a flake form having a specific surface area of 110 to 3000 m²/g, an average particle diameter of 5 to 500µm, an average thickness of 0.10 to 5µm, and an average aspect ratio of 5 to 300.

2. The porous metal oxide material in a flake form according to Claim 1, wherein the porous metal oxide material in a flake form has a peak fine pore diameter of 2 to 20 nm.

3. The porous metal oxide material in a flake form according to Claim 1 or 2, wherein the porous metal oxide material in a flake form is obtained by applying a colloid solution containing colloidal particles of the metal oxide having a particle diameter of 5 to 500 nmon a substrate, drying to solidify the colloid solution, delaminating the resultant solid from the substrate, and heating the solid.

4. The porous metal oxide material in a flake form according to any one of Claims 1 to 3, wherein the porous metal oxide material in a flake form primarily contains at least one kind selected from the group consisting of silicon dioxide (SiO₂), magnesium oxide (MgO), aluminum oxide (Al₂O₃), zirconium oxide (ZrO₂), zinc oxide (ZnO) , chrome oxide (Cr₂O₃), titanium dioxide (TiO₂), antimony trioxide (Sb₂O₃), and iron oxide (Fe₂O₃).

5. The porous metal oxide material in a flake form according to Claim 4, wherein the metal oxide material is silicon dioxide or primarily contains silicon dioxide.

6. A carrier formed by carrying an odorant, a coloring agent, an antibacterial agent or a catalyst in the fine pores of the porous metal oxide material in a flake form according to any one of Claims 1 to 5.

7. A cosmetic comprising the porous metal oxide material in a flake form according to any one of Claims 1 to 5.

8. The cosmetic according to Claim 7, wherein the cosmetic contains the flake form of 0.1-95 % by weight.

9. A cosmetic comprising the carrier according to Claim 6.

10. A coating composition comprising the porous metal oxide material in a flake form according to any one of Claims 1 to 5.

11. A coating composition comprising the carrier according to Claim 6.

12. A resin composition comprising the porous metal oxide material in a flake form according to any one of Claims 1 to 5.

13. A resin composition comprising the carrier according to Claim 6.

14. A resin molded body molded by using the resin composition according to Claim 12 or 13.

15. An ink composition comprising the carrier according to Claim 6.

16. A paper comprising the porous metal oxide material in a flake form according to any one of Claims 1 to 5.

17. A method for producing a porous metal oxide material in a flake form according to any one of Claim 1 or Claim 2, which comprises:
applying a colloid solution containing colloidal particles of the metal oxide having a particle diameter of 5 to 500 nm on a substrate;
drying to solidify the colloid solution;
delaminating the resultant solid from the substrate; and
heating the solid.

18. The method for producing the porous metal oxide material in a flake form according to Claim 11, wherein the porous metal oxide material in a flake form primarily contains at least one kind selected from the group consisting of silicon dioxide (SiO₂), magnesium oxide (MgO), aluminum oxide (Al₂O₃), zirconium oxide (ZrO₂), zinc oxide (ZnO), chrome oxide (Cr₂O₃), titanium dioxide (TiO₂), antimony trioxide (Sb₂O₃), and iron oxide (Fe₂O₃).
